# EUROPÄISCHE PATENTSCHRIFT

(11) **EP 1 267 815 B1**
(45) Veröffentlichungstag und Bekanntmachung des Hinweises auf die Patenterteilung: **02.05.2007**
(21) Anmeldenummer: 01936106.2
(22) Anmeldetag: 20.03.2001
(51) Int. Cl.: A61Q 11/00, A61K 8/11

(54) **ZAHNPFLEGEMITTEL IN PORTIONSKAPSELN**
DENTAL CARE AGENTS PROVIDED IN THE FORM OF SINGLE-PORTION CAPSULES
AGENTS DE SOINS DENTAIRES SOUS FORME DE CAPSULES-PORTIONS

(30) Priorität: 29.03.2000 DE 10015662
(43) Veröffentlichungstag der Anmeldung: 02.01.2003
(73) Patentinhaber: Henkel Kommanditgesellschaft auf Aktien, 40589 Düsseldorf (DE)
(72) Erfinder: WÜLKNITZ, Peter, 42799 Leichlingen (DE); DUSCHEK, Nicole, 40595 Düsseldorf (DE); WALTHER-STANGRECKI, Claudia, 40721 Hilden (DE)
(86) Internationale Anmeldenummer: PCT/EP2001/003163
(87) Internationale Veröffentlichungsnummer: WO 2001/072274

(56) Entgegenhaltungen:
- EP-A- 0 378 956
- EP-A- 0 525 731
- WO-A-99/59535
- US-A- 4 071 614

## Beschreibung

Die Erfindung betrifft Zahnpflegemittel in Form einer Portionskapsel für den einmaligen Gebrauch, die mit einer fließfähigen Zubereitung zur Reinigung und Pflege der Zähne oder der Mundhöhle gefüllt ist.

Zahnpflegemittel in Portionskapseln weisen zahlreiche Vorteile auf. Sie lassen sich in einfachen Vorratsbehältern lagern oder aus speziellen Kapsel-Spendern bequem entnehmen. Das Aufbringen auf eine Zahnbürste entfällt, die Kapseln werden direkt in den Mund eingeführt und zerkaut, dabei wird das Zahnpflegemittel freigesetzt und kann nun, z. B. mit Hilfe einer Bürste, zur Zahnreinigung verwendet werden. Die Vorteile einer solchen Anwendungsform für die Zahnpflege z. B. auf Reisen liegen auf der Hand.

Zahnpasten in Portionskapseln sind daher in der Literatur mehrfach vorgeschlagen worden. EP 0 378 956 A1 und DE 4109518 A1 beschreiben solche Zahnpflegemittel, deren Kapseln aus Gelatine bestehen. WO 98/00418 A1 schlägt ebenfalls eßbare Materialien wie Gelatine oder Agar als Kapselmaterial vor. Gelatine hat aber als Kapselmaterial erhebliche Nachteile und eignet sich kaum für flüssige Zubereitungen mit einem hohen Gehalt an Feuchthaltemitteln wie z. B. Glycerin, Sorbit und Polyethylenglycol. Solche Kapseln verlieren bei längerer Lagerung die Form, sie werden weich oder sogar undicht.

Es bestand daher die Aufgabe, ein Zahnpflegemittel in Form von Portionskapseln zu entwickeln, das eine längere Lagerfähigkeit aufweist, das sich bequem anwenden läßt, dessen Kapsel sich im Mund leicht zerbeißen läßt, sich beim Zähneputzen weitgehend auflöst, gesundheitlich unbedenklich und geschmacklich neutral ist.

Es wurde gefunden, daß sich wasserlösliche Polymere aus der Gruppe der Celluloseether, Polyvinylalkolhol, Polyethylenoxid und Gemische (polymer blends) dieser Polymere wesentlich besser als Kapselmaterial zum Einschluß flüssiger, Feuchthaltemittel von Polyol-Typ enthaltender, Zahnreinigungsmittel eignen.

Gegenstand der Erfindung sind Zahnpflegemittel in Form einer Portionskapsel aus einem wasserlöslichen Material, gefüllt mit einer fließfähigen Zubereitung zur Reinigung und/oder Pflege der Zähne und/oder der Mundhöhle, dadurch gekennzeichnet, daß das Kapselmaterial ganz oder überwiegend aus einem wasserlöslichen Polymeren, ausgewählt aus Celluloseethern, Polyvinylalkohol, Polyethylenoxid und Gemischen davon besteht und die fließfähige Zubereitung wenigstens 50 Gew.-% eines Feuchthaltemittels aus der Gruppe der Glycole, Glycolether und Polyole mit 2-6 C-Atomen, der Polyalkylenglycole und der Gemische davon, wenigstens einen Geschmacksstoff und nicht mehr als 10 Gew.-% Wasser enthält.

Erfindungsgemäß geeignete Kapselmaterialien sind literaturbekannt. So wird z. B. in WO 99/40156 A1 eine Alkylenoxid-Polymerkomposition beschrieben, die sich zur Herstellung von Filmen und Kapseln eignet. Aus EP 714 656 A1 ist ein Kapselmaterial auf Basis von Hydroxypropyl-methylcellulose bekannt. In EP 592 130 A2 ist ein Material für harte Kapseln aus Basis von Hydroxypropyl-methyl-cellulose beschrieben. Aus EP 180 287 A2 schließlich war ein Kapselmaterial auf Basis eines Polymer-Blend aus Celluloseethern und Polyvinylalkohol bekannt.

Erfindungsgemäß besteht das Kapselmaterial ganz oder überwiegend aus Celluloseethern, Polyvinylalkohol und/oder Polyethylenoxiden. Mit "überwiegend" ist gemeint, daß weniger als 10 Gew.-% des Kapselmaterials aus Hilfsmitteln bestehen sollte, die zur Weichmachung oder zur Härtung des Kapselmaterials dienen. Die erfindungsgemäß geeigneten wasserlöslichen Polymeren können entweder zu weichen Kapseln oder zu Hartkapseln verarbeitet werden. Die Herstellung harter Kapseln erfolgt nach bekannten Verfahren, z. B. nach den in EP 714 656 A1 und EP 592 130 A2 beschriebenen Eintauch-Verfahren. Zur Herstellung weicher bzw. flexibler Kapseln werden die wasserlöslichen Polymermaterialien zunächst zu Folien verarbeitet. Die Folienbänder lassen sich dann nach den gleichen Verfahren, wie sie für die Herstellung von Weichgelatine-Kapseln bekannt sind (vgl. W. Fahrig, U. Hofer: Die Kapsel, WVT mbH Stuttgart 1983), zu Kapseln verarbeiten. Dabei werden die Folienbänder z. B. zu taschenartigen Gebilden geformt, in diese wird das Füllgut eingebracht und mit einer zweiten Folie verschlossen. Beispiele für solche Verfahren sind z. B. das Colton- und das Upjohn-Verfahren. Beim Norton-Verkapselungsverfahren erfolgt die Formung der Kapsel zwischen zwei Stanzformwerkzeugen. Im oberen Teil der Form wird die Kapsel aus zwei Folienbändern schlauchartig vorgeformt, über ein Füllröhrchen gefüllt und im unteren Teil wird dann der gefüllte Schlauch durch einen Stanzvorgang zur Kapsel verschlossen.

Das Accogel-Verfahren arbeitet mit einer rotierenden Formwalze und Vakuum, wodurch die Folie in die Form hineingezogen wird. In die Mulde wird das Füllgut dosiert. Durch Aufpressen eines zweiten Folienbandes durch eine zweite Formwalze werden die Kapseln verschlossen und ausgestanzt. In ähnlicher Weise arbeitet auch da sogenannte Rotary-Die-Verfahren, das schon 1933 von R.P. Scherer in Detroit (USA) entwickelt wurde. Dieses Verfahren arbeitet mit zwei gegenläufigen Formwalzen und einem Füllkeil. Durch Verschweißen der unteren und seitlichen Nähte bilden sich zuerst taschenartige Gebilde aus, in die mit Hilfe von Dosierpumpen und feinen Füllkanälen das Füllgut eingepreßt wird. Im Zuge der Rotation der Formwalzen wird dann die Kapsel auch oben verschweißt und nach unten hin ausgestoßen.

Eine für andere Kapselmaterialien angepaßte Verfahrensweise ist in WO 97/35537 A1 (Bioprogress Technology Ltd.) beschrieben. Dabei wird eine Benetzung der Folien mit einem solvatisierenden Lösungsmittel vorgeschlagen, um die Verschweißung bzw. Verklebung des Folienmaterials unter Ausbildung einer dichtschließenden Kapselnaht zu verbessern.

In einer bevorzugten Ausführung der Erfindung besteht das Kapselmaterial ganz oder überwiegend aus Methyl-hydroxypropyl-cellulose. Dieses wird bevorzugt in Form einer Folie mit einer Dicke von 0,2 - 1 mm, insbesondere von 0,08 - 0,2 mm zu Kapseln mit einer entsprechenden Wandstärke und einer Füllmenge von 0,5 - 2 ml verarbeitet.

Die beschriebenen Verfahren der Kapselherstellung aus Folien ermöglichen es auch, zwei Folien von unterschiedlicher Farbe oder Transparenz zur Kapselherstellung zu verwenden. Dies kann z. B. durch kleine Mengen von Farbstoffen oder Pigmenten in dem Kapselmaterial erreicht werden. In einer bevorzugten Ausführung der Erfindung besteht daher das Kapselmaterial aus zwei Halbschalen unterschiedlicher Farbe oder Transparenz.

Die dem Rotary-Die-Verfahren analoge Technologie kann auch in der Weise weiterentwickelt werden, daß man eine dritte Folie als Trennwand zwischen den beiden Folien, die die Kapselwände ausbilden, einführt und auf diese Weise Portionskapseln mit zwei getrennten Kammern erhält. Diese Kammern können mit unterschiedlich zusammengesetzten fließfähigen Zubereitungen gefüllt sein. Diese Zubereitungen können sich nicht nur in Farbe und Transparenz unterschieden, vielmehr kann z. B. eine der Kammern mit einer flüssigen und die andere mit einer fließfähigen, pulverförmigen oder teilchenförmigen Zubereitung gefüllt sein. So kann man z. B. in eine einzelne Portionskapsel zwei Komponenten einbringen, die miteinander nicht verträglich sind, indem man sie in zwei unterschiedlich zusammengesetzte Zubereitungen einarbeitet, die in die getrennten Kammern einer solchen 2-Kammer-Portionskapsel eingefüllt werden.

In einer bevorzugten Ausführung ist die Portionskapsel durch eine Trennwand aus dem gleichen Material zweigeteilt und wenigstens eine der beiden Kammern ist mit einer fließfähigen Zubereitung gefüllt, die wenigstens 50 Gew.-% eines Feuchthaltemittels enthält. Die zweite Kammer ist bei dieser Ausführung bevorzugt mit einer pulverförmigen, gut rieselfähigen Zusammensetzung gefüllt. Diese kann z. B. eine wasserbindende Komponente, z. B. Silicagele enthalten und auf diese Weise die Stabilität der Kapsel gegenüber Luftfeuchtigkeit verbessern. Die zweite Kammer einer solchen 2-Kammer-Portionskapsel kann auch z. B. mit einer Pulverzubereitung gefüllt sein, die hydrolyse-oder oxidationsempfindliche Wirkstoffe wie z. B. Ascorbinsäure, Coenzym Q10 oder Pflanzenwirkstoffe wie z. B. Chamazulen enthält. Auch kann man die pulverförmige Zubereitung in der Form eines Brausepulvers formulieren, also mit einem Gehalt an einem Carbonat- oder Bicarbonatsalz und einer pulverförmigen Säure wie z. B. Citronensäure oder einem wasserlöslichen Hydrogencitrat-Salz. Bei Zutritt von Wasser oder dem Speichel des Mundes kommt es dann zu einem angenehm prickelnden und erfrischenden sensorischen Eindruck im Munde.

Die pulverförmigen Zusammensetzungen enthalten bevorzugt typische Zahnpasten-Abrasivstoffe wie z. B. Calciumcarbonat, Dicalciumphosphate, Kieselsäuren, Aluminiumhydroxid und/oder Aluminiumoxid, Zirkoniumsilikat, Bimssteinpulver oder andere inerte, teilchenförmige Stoffe, die aufgrund ihrer Textur für die Anwendung zum Zähneputzen geeignet sind. Solche Komponenten sind z. B. Cellulosepulver, Kieselgur, Talkum, Schichtsilikate, Kunststoffpulver, Pigmente oder gemahlene Pflanzenteile.

Auch Wirkstoffe, die leicht durch andere Zahnpasteninhaltsstoffe adsorbiert und inaktiviert werden, können bevorzugt in der zweiten Kammer separat formuliert werden, z. B. kationische antibakterielle Wirkstoffe wie Chlorhexidin, Hexetidin und Cetylpyridiniumchlorid.

Schließlich können solche Stoffe, die mit Bestandteilchen der Nachbarkammer unter Bildung feinkristalliner Niederschläge reagieren wie z. B. lösliche Calciumsalze, die dann beim Zähneputzen mit löslichen Fluoriden oder Phosphaten zu feinteiligen Calciumphosphaten oder Calciumfluorphosphaten wie Apatit, Hydroxylapatit oder Fluorapatit reagieren, in der zweiten Kammer enthalten sein.

Das erfindungsgemäße Zahnpflegemittel enthält als fließfähige Zubereitung zur Reinigung und/oder Pflege der Mundhöhle und/oder der Zähne eine weitgehend wasserfreie bzw. wasserarme Zubereitung, die wenigstens 50 Gew.-% eines Feuchthaltemittels aus der Gruppe der wasserlöslichen Glycole, Glycolether oder Polyole mit 2-6 C-Atomen, der Polyalkylenglycole oder eines Gemisches davon enthält.

Als wasserlösliche Glycole eignen sich Ethylenglycol, Propandiole und Butandiole. Geeignete Glycolether sind z. B. Ethylglycol, Ethyldiglycol, Diethylenglycol, Triethylenglycol und Dipropylenglycol. Geeignete Polyole sind z. B. Glycerin, Erythrit, Diglycerin, Xylit, Arabit, Sorbit, Mannit, Dulcit. Als Polyalkylenglycole sind vor allem die Polyethylenglycole und Polypropylenglycole sowie die Anlagerungsprodukte von Ethylenoxid am Propylenglycol oder an Polypropylenglycole geeignet. Es sind dabei besonders solche Polyalkylenglycole geeignet, die ein mittleres Molekulargewicht von nicht mehr als 1000 D aufweisen.

Als weitere obligatorische Komponente der fließfähigen Zubereitung ist wenigstens ein Geschmacksstoff enthalten. Als Geschmacksstoffe sind z. B. Süßungsmittel und/oder Aromaöle geeignet. Als Aromaöle kommen alle für Mund- und Zahnpflegemittel gebräuchlichen natürlichen und synthetischen Aromen in Frage. Natürliche Aromen können sowohl in Form der aus den Drogen isolierten etherischen Öle als auch der aus diesen isolierten Einzelkomponenten verwendet werden. Bevorzugt sollte wenigstens ein Aromaöl aus der Gruppe Pfefferminzöl, Krauseminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte synthetisch erzeugten Komponenten dieser Öle enthalten sein. Die wichtigsten Komponenten der genannten Öle sind z. B. Menthol, Carvon, Anethol, Cineol, Eugenol, Zimtaldehyd, Caryophyllen, Geraniol, Citronellol, Linalool, Salven, Thymol, Terpinen, Terpineol, Methylchavicol und Methylsalicylat. Weitere geeignete Aromen sind z.B. Menthylacetat, Vanillin, Jonone, Linalylacetat, Rhodinol und Piperiton.

Als Süßungsmittel können z. B. Saccharin-Natrium, Acesulfam, Aspartam, NatriumCyclamat, Stevioside, Thaumatin, Sucrose, Lactose, Maltose, Fructose oder Glycyrrhizin eingesetzt werden. Die Geschmacksstoffe können in Mengen von 0,01 bis 2 Gew.-% in der Zubereitung enthalten sein. Die fließfähige Zahnpflegemittelzubereitung ist bevorzugt wasserfrei; kleinere Wasseranteile in der Rezeptur sind jedoch der Stabilität der Kapselmembran nicht abträglich. Der Wassergehalt sollte jedoch einen Anteil von 10 Gew.-% nicht übersteigen.

Zusätzlich zu den genannten obligatorischen Komponenten kann die fließfähige Zubereitung weitere für die Reinigung und Gesunderhaltung der Zähne und des Zahnfleisches nützliche Komponenten enthalten. Als solche zusätzlichen Komponenten sind bevorzugt Poliermittel, Fluorverbindungen oder antimikrobielle Wirkstoffe enthalten. Poliermittel unterstützen die mechanische Reinigung der Zahnoberfläche beim Bürsten der Zähne.

Als Poliermittel eignen sich grundsätzlich alle für Zahnpasten bekannten Reibkörper wie z. B. Kreide, Calciumpyrophosphat, Dicalciumphosphat-dihydrat, Kieselsäuren, Aluminiumhydroxid, Aluminiumoxid, Natriumaluminiumsilikate, organische Polymere und Gemische solcher Reibkörper. In geringen Anteilen von nicht mehr als 1 % können auch stärker abrasive Poliermittel wie Bimssteinmehl oder Zirkoniumsilikat mitverwendet werden. Der Gesamtgehalt an Poliermittel liegt vozugsweise im Bereich von 5-30 Gew.-% der fließfähigen Zubereitung.

Bevorzugt geeignet für die Zahnpflegemittel der vorliegenden Erfindung sind Poliermittel vom Typ der Kieselsäuren. Geeignete Kieselsäuren sind z. B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalt von 15 bis 35 Gew.-%, so werden die sogenannten Hydrogelkieselsäuren erhalten, wie sie z. B. aus US 4,153,680 bekannt sind. Durch Trocknung auf Wassergehalte unterhalb 15 Gew.-% erfolgt eine irreversible Schrumpfung der vorher lockeren Struktur des Hydrogels zur dichten Struktur des sog. Xerogels. Solche Xerogelkieselsäuren sind z. B. in US 3,538,230 beschrieben.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Herstellung von Fällungskieselsäuren sind z. B. in DE-OS 25 22 486 und in DE-OS 31 14 493 beschrieben. Bevorzugt geeignet ist eine gemäß DE-OS 31 14 493 hergestellte Fällungskieselsäure mit einer BET-Oberfläche von 15-110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident^{®}12 DS (DEGUSSA) erhältlich.

Andere Fällungskieselsäuren dieser Art sind Sident 8 (DEGUSSA) und Sorbosil AC 39 (Crosfield Chemicals). Diese Kieselsäuren zeichnen sich durch eine geringere Verdickungswirkung und eine etwas höhere mittlere Teilchengröße von 8 -14 µm bei einer spezifischen Oberfläche von 40 - 75 m²/g (nach BET) aus und eignen sich besonders gut für flüssige Zubereitungen.

Zubereitungen, die eine höhere Viskosität aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zubereitungen setzt man daher bevorzugt neben den genannten Fällungskieselsäuren noch feinteiligere, sogenannte Verdickungs-kieselsäuren mit einer BET-Oberfläche von 150 - 250 m²/g zu, z. B. die Handelsprodukte Sipernat 22 LS oder Sipernat 320 DS.

Als weitere Poliermittelkomponente kann auch z. B. Aluminiumoxid in Form von schwach calcinierter Tonerde mit einem Gehalt an γ- und α-Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich. Fluorverbindungen dienen der Härtung des Zahnschmelzes und damit der Kariesprophylaxe.

Als Fluorverbindungen können die erfindungsgemäßen Zahnpflegemittel z. B. Natriumfluorid, Zinkfluorid, Zinn-(II)-fluorid, Aminfluorid oder Natriummonofluorophosphat enthalten. Bevorzugt sollte eine Menge von 0,01 - 0,2 Gew.-% Fluor in Form der genannten Verbindungen enthalten sein.

Antimikrobielle Verbindungen sind wirksam gegen die Protein und Stärke abbauenden und säurebildenden Bakterien der Zahnplaque und gegen die besonders hartnäckigen Keime der chronischen Zahnfleischentzündungen. Sie beugen daher der Bildung von Mundgeruch, Karies und Parodontitis vor. Geeignete antimikrobielle Verbindungen sind z. B. kationische oberflächenaktive Stoffe wie z. B. Cetyltrimethylammoniumbromid, Benzethoniumchlorid, Cetylpyridiniumchlorid oder das als Aminfluorid bekannte N,N,N'-tris-(2-Hydroxyethyl)-N'-octadecyl-1,3-diaminopropan-dihydrofluorid. Gut eignen sich auch die antimikrobiell wirksamen Biguanidverbindungen wie z. B. das Polyhexamethylenbiguanid (Vantocil^{®} IB, ICJ) oder das 1,1'-Hexamethylen-bis-(4-Chlorphenyl)-biguanid ("Chlorhexidin") in Form eines wasserlöslichen, verträglichen Salzes, z. B. in Form des Acetats oder Gluconats. Bevorzugt eignen sich auch die antimikrobiellen 5-Amino-hexahydropyrimidine, z. B. das 1,3-Bis-(2-ethylhexyl)-5-methyl-5-amino-hexahydropyrimidin ("Hexetidin"). Weitere bevorzugt geeignete antimikrobielle Wirkstoffe sind die nichtkationischen, phenolischen, antimikrobiellen Stoffe, insbesondere die halogenierten Phenole und Diphenylether. Besonders geeignete antimikrobielle Verbindungen dieses Types sind z. B. das 6,6'- Methylen-Bis-(2-brom-4-chlorphenol) ("Bromchlorophen") und der 2,4,4'-Trichlor-2'-hydroxy-diphenylether ("Triclosan").

Weitere geeignete antimikrobielle Stoffe sind die p-Hydroxybenzoesäureester und Sesquiterpenalkohole wie z. B. das Bisabolol, das Farnesol, das Santalol oder das Nerolidol. Antimikrobielle Wirkstoffe können in den erfindungsgemäßen Zahnpflegemitteln in einer Menge von 0,005 bis 0,5 Gew.-% der fließfähigen Zubereitung enthalten sein.

Neben diesen bevorzugt enthaltenen Komponenten können die erfindungsgemäßen Zahnpflegemittel auch weitere, auf dem Gebiet der Zahnpflegemittel, bekannten Wirkstoffe und Hilfsmittel enthalten. Solche geeigneten Wirkstoffe sind z. B.
- wundheilende und entzündungshemmende Stoffe wie z. B. Allantoin, Harnstoff, Azulen, Kamillenwirkstoffe, Acetylsalicylsäurederivate
- Vitamine wie z. B. Retinol oder Retinolester, Ascorbinsäurederivate, Panthenol, Biotin.
- Desensibilisierende Komponenten wie z. B. Kalium- oder Strontiumsalze, Eugenol, Nelkenknospenöl
- Remineralisierende Salze wie z. B. Calcium- und Magnesiumsalze, insbesondere Phosphate.
- Antizahnsteinwirkstoffe wie z. B. kondensierte Phosphate wie z. B. Na-Pyrophosphat, Na-Tripolyphosphat, Organophosphonate wie z. B. das Natriumsalz der 1-Hydroxyethan-1,1-diphosphonsäure, der 1-Phosphonopropan-1,2,3-triphosphonsäure oder der Azacycloheptan-2,2-diphosphonsäure, Phosvitin, Tranexamsäure und andere Komplexbildner

Geeignete Hilfsmittel zur Einstellung der Konsistenz oder des pH-Wertes, der Farbe und Transparenz sind z. B.
- Bindemittel wie z. B. Cellulose, Celluloseether, Stärke und Stärkeether, Biopolymere wie z. B. Xanthan-Gum, Pflanzengummi wie z. B. Agar-Agar, Carragheenan, Traganth, Guar, Akaziengummi, Johannisbrotkernmehl, Pektine und synthetische Polymere wie z. B. Polyvinylpyrrolidon, Polyvinylalkohol und Carboxyvinylpolymere.
- Anorganische Verdickungsmittel wie z. B. kolloidale Kieselsäure (Aerogel-Kieselsäure, pyrogene Kieselsäuren), Schichtsilikate (Tone, Montmorillonit),
- Farbstoffe, Pigmente, z. B. Titandioxid
- Puffersubstanzen wie z. B. Citronensäure-/Natriumcitrat oder Gemische aus primären, sekundären oder tertiären Alkaliphosphaten

Zur Verbesserung des Reinigungsvermögens und zur stabilen Emulgierung oder Solubilisierung der Geschmacksstoffe können außerdem Tenside und niedere Alkohole in kleineren Mengen von insgesamt nicht mehr als 3 Gew.-% enthalten sein.

Der Tensidzusatz kann auch zur Erzeugung eines Schaums beim Zähnebürsten, zur Stabilisierung der Poliermitteldispersion sowie zur Emulgierung oder Solubilisierung der Aromaöle erwünscht sein. Geeignete Tenside, die eine gewisse Schaumwirkung entfalten, sind die anionischen Tenside, z. B. Natriumalkylsulfate mit 12 - 18 C-Atomen in der Alkylgruppe. Diese Tenside weisen auch eine gewisse enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12 - 16 C-Atomen in der linearen Alkylgruppe und 2 - 6 Glycolethergruppen im Molekül, von linearem Alkan-(C₁₂-C₁₈)-sulfonat, von Sulfobernsteinsäuremonoalkyl-(C₁₂-C₁₈)-estern, von sulfatierten Fettsäuremonoglyceriden, sulfatierten Fettsäurealkanolamiden, Sulfoessigsäurealkyl-(C₁₂-C₁₆)-estern, Acylsarcosinen, Acyltauriden und Acylisethionaten mit jeweils 8 -18 C-Atomen in der Acylgruppe.

Auch zwitterionische und ampholytische Tenside können, bevorzugt in Kombination mit anionischen Tensiden, eingesetzt werden. Besonders bevorzugt zur Förderung der Reinigungswirkung ist aber der Einsatz nichtionogener Tenside. Geeignete nichtionische Tenside sind z. B. die Anlagerungsprodukte von Ethylenoxid an Fettalkohole, an Fettsäuren, an Fettsäuremonoglyceride, an Sorbitan-Fettsäuremonoester oder an Methylglucosid-Fettsäuremonoester. Die angelagerte Menge an Ethylenoxid sollte dabei so hoch sein, daß die Tenside wasserlöslich sind, d.h. es sollte wenigstens 1 g/l in Wasser bei 20°C löslich sein. Eine weitere Gruppe von geeigneten Tensiden sind die Alkyl-(oligo)-glycoside mit 8 - 16 C-Atomen in der Alkylgruppe und einem Oligomerisationsgrad des Glycosidrestes von 1 - 4. Alkyl-(oligo)-glycoside, ihre Herstellung und Verwendung als oberflächenaktive Stoffe sind z. B. aus US-A-3,839,318, DE-A-20 36 472, EP-A-77 167 oder WO-A-93/10132 bekannt.

Bezüglich des Glycosidrestes gilt, daß sowohl Monoglycoside (x = 1), bei denen ein Monosaccharidrest glycosidisch an einen Fettalkohol mit 10 bis 16 C-Atomen gebunden ist, als auch oligomere Glycoside mit einem Oligomerisationsgrad x bis 10 geeignet sind. Der Oligomerisationsgrad ist dabei ein statistischer Mittelwert, dem eine für solche technischen Produkte übliche Homologenverteilung zugrunde liegt.

Bevorzugt eignet sich als Alkyl-(oligo)-glycosid ein Alkyl-(oligo)-glucosid der Formel RO(C₆H₁₀O)ₓ-H, in der R eine Alkylgruppe mit 12 bis 14 C-Atomen ist und x einen Mittelwert von 1 bis 4 hat.

Insbesondere zur Solubilisierung der meist wasserunlöslichen Aromaöle kann ein nichtionogener Lösungsvermittler aus der Gruppe der oberflächenaktiven Verbindungen erforderlich sein. Besonders geeignet für diesen Zweck sind z. B. oxethylierte Fettsäureglyceride, oxethylierte Fettsäure-sorbitanpartialester oder Fettsäurepartialester von Glycerin- oder Sorbitan-Oxethylaten. Lösungsvermittler aus der Gruppe der oxethylierten Fettsäureglyceride umfassen vor allem Anlagerungsprodukte von 20 bis 60 Mol Ethylenoxid an Mono- und Diglyceride von linearen Fettsäuren mit 12 bis 18 C-Atomen oder an Triglyceride von Hydroxyfettsäuren wie Oxystearinsäure oder Ricinolsäure. Weitere geeignete Lösungsvermittler sind oxethylierte Fettsäuresorbitanpartialester; das sind bevorzugt Anlagerungsprodukte von 20 bis 60 Mol Ethylenoxid an Sorbitanmonoester und Sorbitandiester von Fettsäuren mit 12 bis 18 C-Atomen. Ebenfalls geeignete Lösungsvermittler sind Fettsäurepartialester von Glycerin- oder Sorbitan-Oxethylaten; das sind bevorzugt Mono- und Diester von C₁₂-C₁₈-Fettsäuren und Anlagerungsprodukten von 20 bis 60 Mol Ethylenoxid an 1 Mol Glycerin oder an 1 Mol Sorbit. Als niedere Alkohole können 0,1-2Gew.-% Ethanol oder Isopropanol enthalten sein.

Die folgenden Beispiele sollen den Erfindungsgegenstand näher erläutern:

### Beispiele

1. Zahncreme-Formulierungen für MHPC-Kapseln

| | 1.1 | 1.2 |
|---|---|---|
| Glycerin (99,5 %-ig) | 40,0 | 93,7 |
| Sorbitol (100 %-ig) | 15,0 | ---- |
| 1,2-Propylenglycol | 17,0 | ---- |
| Polyethylenglycol 400 | 2,0 | 3,0 |
| Keltrol F | 0,2 | 0,9 |
| Sident 8 | 12,0 | ---- |
| Sident 22 S | 4,5 | ---- |
| Kieselsäure FK 320 DS | 1,0 | ---- |
| Saccharin-Na | 0,25 | ---- |
| Aromaöl | 0,1 | 0,1 |
| Tagat S | 0,1 | 0,1 |
| Na-Dihydrogenphosphat | ---- | 0,2 |
| Na-Dihydrogencitrat | ---- | 0,2 |
| Farbstoff (grün), Ci 74260 | ---- | 0,01 |
| Wasser | ad 100 | ad100 |

Die Zusammensetzungen wurden nach dem in WO 97/35537 A1 beschriebenen Verfahren in Portionskapseln aus Methyl-hydroxypropylcellulose mit einem Inhalt 1 ml und einer Wandstärke von 0,1 mm abgefüllt. Die erhaltenen Kapseln waren bei trockener Lagerung bei 25°C nach 6 Wochen unverändert.
Es wurden folgende Handelsprodukte verwendet:

| | |
|---|---|
| Keltrol F (Kelco): | Xanthan Gum |
| Sident 8 (Degussa): | Synth. amorphe Kieselsäure, BET: 60 m²/g |
| Sident 22 S (Degussa): | Synth. amorphe Kieselsäure, BET: 140 m²/g |
| Kieselsäure FK 320 DS (Degussa): | Synth. amorphe Kieselsäure, BET: 170 m²/g |
| Tagat S (Tego Cosmet): | PEG 30 Glyceryl Stearate |

2. Pulver-Formulierung für eine Zweikammer-Portionskapsel
Die erste Kammer enthält eine Zahncreme gemäß Beispiel 1.1 oder 1.2. Die zweite Kammer enthält eine Pulver-Zusammensetzung gemäß Rezeptur 2.1, 2.2 oder 2.3.

| | 2.1 | 2.2 | 2.3 |
|---|---|---|---|
| Calciumcarbonat | 95,0 | ---- | ---- |
| Dicalciumphosphat-dihydrat | ---- | ---- | 98,2 |
| Cellulosepulver | ---- | 99,0 | ---- |
| Natriumhydrogencarbonat | 5,0 | ---- | ---- |
| Natriumhydrogenphosphat | ---- | ---- | 1,0 |
| Natriumdihydrogenphosphat | ---- | ---- | 0,5 |
| Natriumfluorid | ---- | ---- | 0,3 |
| Kamille, pulverisiert | ---- | 1,0 | ---- |

## Patentansprüche

1. Zahnpflegemittel in Form einer Portionskapsel aus einem wasserlöslichen Material, gefüllt mit einer fließfähigen Zubereitung zur Reinigung und/oder Pflege der Mundhöhle und/oder der Zähne, **dadurch gekennzeichnet, daß** das Kapselmaterial ganz oder überwiegend aus einem wasserlöslichen Polymeren, ausgewählt aus Celluloseethern, Polyvinylalkohol, Polyethylenoxid und Gemischen davon besteht und die fließfähige Zubereitung wenigstens 50 Gew.-% eines Feuchthaltemittels, ausgewählt aus der Gruppe der Glycole, Glycolether oder Polyole mit 2-6 C-Atomen, der Polyalkylenglycole oder eines Gemisches davon, wenigstens einen Geschmacksstoff und nicht mehr als 10 Gew.-% Wasser enthält.

2. Zahnpflegemittel nach Anspruch 1, **dadurch gekennzeichnet, daß** das Kapselmaterial ganz oder überwiegend aus einer Methyl-Hydroxypropyl-Cellulose besteht.

3. Zahnpflegemittel nach einem der Ansprüche 1 oder 2, **dadurch gekennzeichnet, daß** das Kapselmaterial aus zwei Halbschalen unterschiedlicher Transparenz oder Farbe besteht.

4. Zahnpflegemittel nach einem der Ansprüche 1 oder 2, **dadurch gekennzeichnet, daß** die Portionskapsel durch eine Trennwand aus dem gleichen Material zweigeteilt ist und wenigstens eine der beiden Kammern mit einer fließfähigen Zubereitung, gemäß Anspruch 1, gefüllt ist.

5. Zahnpflegemittel nach Anspruch 4, **dadurch gekennzeichnet, daß** die zweite Kammer mit einer fließfähigen Pulverzusammensetzung gefüllt ist.

6. Zahnpflegemittel gemäß einem der Ansprüche 2-4, **dadurch gekennzeichnet, daß** die fließfähige Zubereitung zusätzlich Poliermittel, Fluorverbindungen und/oder antimikrobielle Wirkstoffe enthält.

## Claims

1. Dental care agent in the form of a portion capsule of a water-soluble material filled with a flowable preparation for cleaning and/or care of the oral cavity and/or the teeth, **characterized in that** the capsule material consists wholly or predominantly of a water-soluble polymer selected from the group consisting of cellulose ethers, polyvinyl alcohol, polyethylene oxide and mixtures thereof and the flowable preparation contains at least 50% by weight of a humectant selected from the group consisting of glycols, glycol ethers or polyols having 2-6 carbon atoms, polyalkylene glycols or a mixture thereof, at least one flavouring agent and not more than 10% by weight of water.

2. Dental care agent according to Claim 1, **characterized in that** the capsule material consists wholly or predominantly of a methyl hydroxypropyl cellulose.

3. Dental care agent according to Claim 1 or Claim 2, **characterized in that** the capsule material consists of two half shells differing in transparency or colour.

4. Dental care agent according to Claim 1 or Claim 2, **characterized in that** the portion capsule is divided in two by a partition of the same material and at least one of the two compartments is filled with a flowable preparation as defined in Claim 1.

5. Dental care agent according to Claim 4, **characterized in that** the second compartment is filled with a flowable pulverulent composition.

6. Dental care agent according to any one of the Claims 2-4, **characterized in that** the flowable preparation additionally contains polishing agents, fluorine compounds and/or antimicrobially active compounds.

## Revendications

1. Produit de soins dentaires sous forme de capsule-portion qui est constituée d'une matière hydrosoluble et remplie d'une préparation fluide destinée au nettoyage et/ou aux soins de la cavité buccale et/ou des dents, **caractérisé en ce que** la matière de la capsule est constituée, en totalité ou en majeure partie, par un polymère hydrosoluble choisi parmi les éthers cellulosiques, l'alcool polyvinylique, le poly(oxyde d'éthylène) et les mélanges de ces composés, et la préparation fluide contient au moins 50 % en poids d'un agent de maintien de l'humidité, choisi dans le groupe constitué par les glycols, les éthers de glycol ou les polyols comprenant 2 à 6 atomes de C, les polyalkylèneglycols ou un mélange de ces composés, au moins un agent de goût et au maximum 10 % en poids d'eau.

2. Produit de soins dentaires selon la revendication 1, **caractérisé en ce que** la matière de la capsule est constituée, en totalité ou en majeure partie, par une méthyl-hydroxypropyl-cellulose.

3. Produit de soins dentaires selon l'une des revendications 1 ou 2, **caractérisé en ce que** la matière de la capsule est constituée de deux demi-coquilles qui présentent chacune une transparence ou une coloration différente.

4. Produit de soins dentaires selon l'une des revendications 1 ou 2, **caractérisé en ce que** la capsule-portion est séparée en deux au moyen d'une paroi constituée par la même matière que la capsule, et au moins un des deux compartiments est rempli avec une préparation liquide selon la revendication 1.

5. Produit de soins dentaires selon la revendication 4, **caractérisé en ce que** le second compartiment est rempli d'une composition pulvérulente fluide.

6. Produit de soins dentaires selon l'une quelconque des revendications 2 à 4, **caractérisé en ce que** la préparation fluide contient en outre un agent de polissage, des composés fluorés et/ou des principes actifs antimicrobiens.
